# EUROPEAN PATENT SPECIFICATION

(11) **EP 1 580 275 B1**
(45) Date of publication and mention of the grant of the patent: **02.09.2009**
(21) Application number: 05102204.4
(22) Date of filing: 18.03.2005
(51) Int. Cl.: C12N 15/82, C12N 15/29, C07K 14/415

(54) **Plants having increased seed yield and method for making the same**
Pflanzen mit erhöhtem Saatertrag und Verfahren zu deren Herstellung
Plantes ayant un rendement d'ensemencement accru et procédé de fabrication desdites plantes

(30) Priority: 22.03.2004 EP 04101179; 26.03.2004 US 556847 P
(43) Date of publication of application: 28.09.2005
(73) Proprietor: CropDesign N.V., 9052 Zwijnaarde (BE)
(72) Inventor: Frankard, Valerie, Dr., 1180 Bruxelles (BE); Dudits, Denes, Dr., 6726 Szeged (HU); Feher, Attila, Dr., 6771 Szeged (HU)
(74) Representative: Mistry, Meeta

(56) References cited:
- EP-A- 1 033 405
- WO-A-00/56905
- KODAMA H ET AL: "ISOLATION OF GENES THAT ARE PREFERENTIALLY EXPRESSED AT THE G-1-S BOUNDARY DURING THE CELL CYCLE IN SYNCHRONIZED CULTURES OF CATHARANTHUS-ROSEUS CELLS" PLANT PHYSIOLOGY (ROCKVILLE), vol. 95, no. 2, 1991, pages 406-411, XP002330934 ISSN: 0032-0889
- LYAMOURI M ET AL: "Organization, sequence, and phylogenetic analysis of the ribosomal protein S3 gene from Drosophila virilis" GENE: AN INTERNATIONAL JOURNAL ON GENES AND GENOMES, ELSEVIER SCIENCE PUBLISHERS, BARKING, GB, vol. 294, no. 1-2, 10 July 2002 (2002-07-10), pages 147-156, XP004381347 ISSN: 0378-1119

## Description

The present invention relates generally to the field of molecular biology and concerns a method for increasing seed yield, by transforming a plant with a gene construct comprising a nucleic acid encoding a 40S small subunit ribosomal protein (S3a). The present invention also concerns plants having introduced therein an S3a-encoding nucleic acid, which plants have improved seed yield relative to corresponding wild type plants.

The ever-increasing world population and the dwindling supply of arable land available for agriculture fuel agricultural research towards improving the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits. A trait of particular economic interest is yield. Yield is normally defined as the measurable produce of economic value from a crop. This may be defined in terms of quantity and/or quality. Yield is directly dependent on several factors, for example, the number and size of the organs, plant architecture (for example, the number of branches), seed production and more. Root development, nutrient uptake and stress tolerance are also important factors in determining yield. Crop yield may be increased by optimizing one of the abovementioned factors.

An outline of the ribosome assembly pathway emerged from sucrose gradient analyses in the early 1970s. These studies identified three major pre-ribosomal particles. An early 90S particle ('S' being the rate of sedimentation) is subsequently processed into 66S and 43S preribosomes, the precursors to the mature 60S and 40S subunits, respectively. The 60S subunit, after processing, is composed of 255, 5.8S and 5S ribosomal RNA. A number of proteins will have interacted along the maturation process to yield the final structure. The 40S subunit is composed of an 18S ribosome RNA, and again a number of proteins will have interacted before the mature complex is ready. These two subunits assemble into a large complex in which the small subunit 40S binds the mRNA and the tRNAs, while the large subunit catalyzes peptide bond formation. About 2/3 of the mass of the ribosome consists of RNA and 1/3 of protein. The proteins are named in accordance with the subunit of the ribosome to which they belong: the small subunit (S1 to S31) and the large subunit (L1 to L44). Most of the proteins interact with multiple RNA elements, often from different domains, to organize and stabilize the rRNA tertiary structure. While the crucial activities of decoding and peptide transfer are RNA based processes, proteins play an active role in functions that may have evolved to streamline the process of protein synthesis. In addition to their typical ribosomal functions, many ribosomal proteins also have non-ribosomal functions, such asDNA repair.

S3a (cyc07) was first cloned in 1991. It encodes a 40S ribosomal protein (small subunit) and is homologous to the v-fos transformation effector (encoded by the fte-1 gene in humans and rats), the TNF-alpha-induced TU-11 gene in mice, and is similar to the yeast PLC1 and PLC2. The expressions of mammalian fte-1, plant cyc-07 and yeast PLC2 have all been shown to be cell-cycle-regulated and involved in protein synthesis at the level of the ribosome.

The ability to improve various growth characteristics of a plant, would have many applications in areas such as crop enhancement, plant breeding, in the production of ornamental plants, aboriculture, horticulture, forestry, the production of algae for use in bioreactors (for the biotechnological production of substances such as pharmaceuticals, antibodies, or vaccines, or for the bioconversion of organic waste) and other such areas.

It has now been found that transforming a plant with a gene construct comprising a nucleic acid encoding a 40S small subunit ribosomal protein (S3a) gives plants having increased seed yield relative to corresponding wild type plants. Therefore according to one embodiment of the present invention there is provided a method for increasing seed yield in a plant, comprising transforming a plant with a gene construct comprising a nucleic acid encoding a 40S small subunit ribosomal protein (S3a).

Advantageously, performance of the methods according to the present invention result in plants having increased seed yield..

The term "increased yield" as defined herein is taken to mean an increase in any one or more of the following, each relative to corresponding wild type plants: (i) increased biomass (weight) of one or more parts of a plant, particularly aboveground (harvestable) parts, increased root biomass or increased biomass of any other harvestable part; (ii) increased seed yield, which may result from an increase in seed biomass (seed weight) and which may be an increase in the seed weight per plant or on an individual seed basis, and which increase in seed weight may be due to altered seed dimensions, such as seed length and/or seed width and/or seed area; (iii) increased number of (filled) seeds; (iv) increased seed size, which may also influence the composition of seeds; (v) increased seed volume, which may also influence the composition of seeds; (vi) increased harvest index, which is expressed as a ratio of the yield of harvestable parts, such as seeds, over the total biomass; and (vii) increased thousand kernel weight (TKW), which is extrapolated from the number of filled seeds counted and their total weight. An increased TKW may result from an increased seed size and/or seed weight.

According to the invention, the increase in yield encompasses an increase in yield on a seed level as defined in any one or more of (ii) to (vii) above.

Taking com as an example, a yield increase may be manifested as one or more of the following: increase in the number of plants per hectare or acre, an increase in the number of ears per plant, an increase in the number of rows, number of kernels per row, kernel weight, thousand kernel weight, ear length/diameter, among others. Taking rice as an example, a yield increase may be manifested by an increase in one or more of the following: number of plants per hectare or acre, number of panicles per plant, number of spikelets per panicle, number of flowers per panicle, increase in the seed filling rate, increase in thousand kernel weight, among others. An increase in yield may also result in modified architecture, or may occur as a result of modified architecture.

According to a preferred feature of the present invention, performance of the methods of the invention result in plants having increased yield which is manifested by at least one of: increased TKW, increased number of (filled) seeds, increased seed weight and increased harvest index, each relative to control plants. Therefore, according to the present invention, there is provided a method for increasing plant seed yield, which method comprises transforming a plant with a gene construct comprising a nucleic acid encoding a 40S small subunit ribosomal protein (S3a polypeptide).

Since the transgenic plants according to the present invention have increased seed field, it is lively that these plants exhibit an increased growth rate (during at least part of their life cycle), relative to the growth rate of corresponding wild type plants at a corresponding stage in their life cycle. The increased growth rate may be specific to one or more parts of a plant (including seeds), or may be throughout substantially the whole plant. A plant having an increased growth rate may even exhibit early flowering. The increase in growth rate may take place at one or more stages in the life cycle of a plant or during substantially the whole plant life cycle.

Increased growth rate during the early stages in the life cycle of a plant may reflect enhanced vigour, The increase in growth rate may alter the harvest cycle of a plant allowing plants to be sown later and/or harvested sooner than would otherwise be possible. If the growth rate is sufficiently increased, it may allow for the sowing of further seeds of the same plant species (for example sowing and harvesting of rice plants followed by sowing and harvesting of further rice plants all within one conventional growing period). Similarly, if the growth rate is sufficiently increased, it may allow for the sowing of further seeds of different plants species (for example the sowing and harvesting of rice plants followed by, for example, the sowing and optional harvesting of soy bean, potatoes or any other suitable plant). Harvesting additional times from the same rootstock in the case of some plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves plotting growth experiments, such parameters may be: T-Mid (the time taken for plants to reach 50% of their maximal size) and T-90 (time taken for plants to reach 90% of their maximal size), amongst others.

Performance of the methods of the invention gives plants having an increased growth rate. Therefore, according to the present invention, there is provided a method for increasing the growth rate of plants, which method comprises transforming a plant with a gene construct comprising a nucleic acid encoding a 40S small subunit ribosomal protein (S3a polypeptide). An increase in growth rate is exemplified herein by an increase in TKW, increased number of (filled) seeds, increased seed weight and increased harvest index, each relative to control plants/corresponding wild-type plants.

An increase in seed yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the typical stresses to which a plant may be exposed. These stresses may be the everyday biotic and/or abiotic (environmental) stresses to which a plant is exposed. Typical abiotic or environmental stresses include temperature stresses caused by atypical hot or cold/freezing temperatures; salt stress; water stress (drought or excess water). Abiotic stresses may also be caused by chemicals. Biotic stresses are typically those stresses caused by pathogens, such as bacteria, viruses, fungi and insects.

The abovementioned growth characteristics may advantageously be modified in any plant.

The term "plant" as used herein encompasses whole plants, ancestors and progeny of the plants and plant parts, including seeds, shoots, stems, leaves, roots (including tubers), and tissues and organs, wherein each of the aforementioned comprise the gene of interest. The term "plant" also encompasses embryos, meristematic regions, gametophyles, sporophytes, pollen, and microspores, again wherein each of the aforementioned comprise the gene of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acacia* spp., Acer spp., *Actinidia* spp., *Aesculus* spp., *Agathis australis, Albizia amara, Alsophila tricolor, Andropogon* spp., *Arachis* spp, *Areca catechu, Astelia fragrans, Astragalus cicer, Baikiaea plurijuga, Betula* spp., *Brassica* spp., *Bruguiera gymnorrhiza, Burkea africana, Butea frondosa, Cadaba farinosa, Calliandra* spp, *Camellia sinensis, Canna indica, Capsicum* spp., Cassia spp., *Centroema pubescens, Chaenomeles* spp., *Cinnamomum cassia, Coffea arabica, Colophospermum mopane, Coronillia varia, Cotoneaster serotina, Crataegus* spp., *Cucumis* spp., *Cupressus* spp., *Cyathea dealbata, Cydonia oblonga, Cryptomeria japonica, Cymbopogon* spp., *Cynthea dealbata, Cydonia oblongs, Dalbergia monetaria, Davallia divaricata, Desmodium* spp., *Dicksonia squarosa. Diheteropogon amplectens, Dioclea* spp, *Dolichos* spp., *Dorycnium rectum, Echinochloa pyramidalis, Ehrartia* spp., *Eleusine coracana, Eragrestis* spp., *Erythrina spp., Eucalyptus* spp., *Euclea schimperi, Eulalia villosa, Fagopyrum* spp., *Feijoa sellowiana, Fragaria* spp., *Flemingia* spp, *Freycinetia banksii, Geranium thunbergii, Ginkgo biloba, Glycine javanica, Gliricidia* spp, *Gossypium hirsutum, Grevillea* spp., *Guibourtia coleosperma, Hedysarum* spp., *Hemarthia altissima, Heteropogon contortus, Hordeum vulgare, Hyparrhenia rufa, Hypericum erectum, Hyperthelia dissoluta, Indigo incarnata, Iris spp., Leptarrhena pyrolifolia, Lespediza* spp., *Lettuca* spp., *Leucaena leucocephala, Loudetia simplex, Lotonus bainesii, Lotus* spp., *Macrotyloma axillare, Malus* spp., *Manihot esculenta, Medicago sativa, Metasequoia glyptostroboides, Musa sapientum, Nicotianum* spp., *Onobrychis* spp., *Omithopus* spp., *Oryza* spp., *Peltophorum africanum, Pennisetum* spp., *Persea gratissima, Petunia* spp., *Phaseolus* spp., *Phoenix canariensis, Phormium cookianum, Photinia* spp., *Picea glauca, Pinus* spp., *Pisum sativum, Podocarpus totara, Pogonarthria fleckii, Pogonarthria squarrosa, Populus* spp., *Prosopis cineraria, Pseudolsuga menziesii, Pterolobium stellatum, Pyrus communis, Quercus* spp., *Rhaphiolopsis umbellata, Rhopalostylis sapida, Rhus natalensis, Ribes grossularia, Ribes* spp., *Robinia pseudoacacia, Rosa* spp., *Rubus* spp., *Salix* spp., *Schyzachyrium sanguineum, Sciadopitys verticillata, Sequoia sempervirens, Sequoiadendron giganteum, Sorghum bicolor, Spinacia* spp., *Sporobolus fimbriatus, Stiburus alopecuroides, Stylosanthos humilis, Tadehagi* spp, *Taxodium distichum, Themeda triandra, Trifolium* spp., *Triticum* spp., *Tsuga heterophylla, Vaccinium* spp., *Vicia* spp., *Vitis vinifera, Watsonia pyramidata, Zantedeschia aethiopica, Zea mays,* amaranth, artichoke, asparagus, broccoli, Brussels sprouts, cabbage, canola, carrot, cauliflower, celery, collard greens, flax, kale, lentil, oilseed rape, okra, onion, potato, rice, soybean, strawberry, sugar beet, sugar cane, sunflower, tomato, squash, tea and algae, amongst others. According to a preferred embodiment of the present invention, the plant is a crop plant such as soybean, sunflower, canola, alfalfa, rapeseed, cotton, tomato, potato or tobacco. Further preferably, the plant is a monocotyledonous plant, such as sugarcane. More preferably the plant is a cereal, such as rice, maize, wheat, barley, millet, rye, sorghum or oats.

The term S3a is well known in the art. Alternative names for S3a are given in Table 1 below taken from Naora and Naora (Immunology and Cell Biology (1999) 77, 197-205).

**Table 1: Homologues of S3a and Nomenclature**

| Name | Species | Identifying criterion | Reference |
|---|---|---|---|
| *RPS3a* | Human | Ribosomal protein | Metspalu et a/., 1992 |
| *nbl* | Human | Abundant expression in Namalwa Burkitt Lymphoma cells | Naora *et al*., 1995 |
| *RPS3a* | Cat | Abundant expression in feline leukaemia virus-induced lymphomas | Starkey and Levy, 1995 |
| *fte-1* | Rat | Disruption in revertant v-fos-transformed fibroblasts | Kho and Zarbl, 1992 |
| *TU-11* | Mouse | Expression induced by TNF-a | Gordon *et al.,* 1992 |
| *13T* | Mouse | Rat hybrid cells Abundant expression in transformed hybrids | Lecomte *et al.,* 1997 |
| *KRP-A* | Sea hare | Abundant expression in neurons | Auclair *et al.,* 1994 |
| *C3* | Mosquito | Preferential expression in ovary | Zurita *et al.,* 1997 |
| *RPS3a* | Fruit fly | Mutation associated with Minute phenotype | van Beest *et al.,* 1998 |
| *cyc07* | Higher plant | S-phase-specific expression | Ito *et al.,* 1991 |
| *MFT1* | Yeast | Mutation impairs protein import into mitochondria | Garrett *et al.,* 1991 |

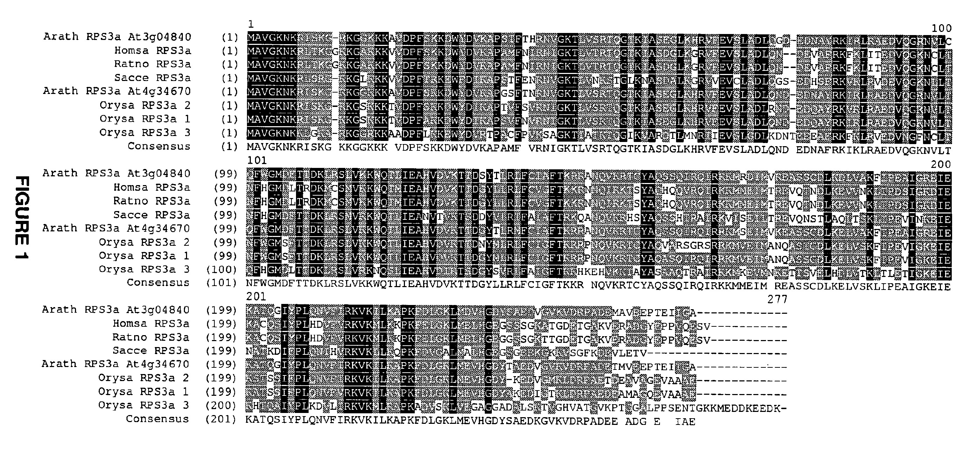

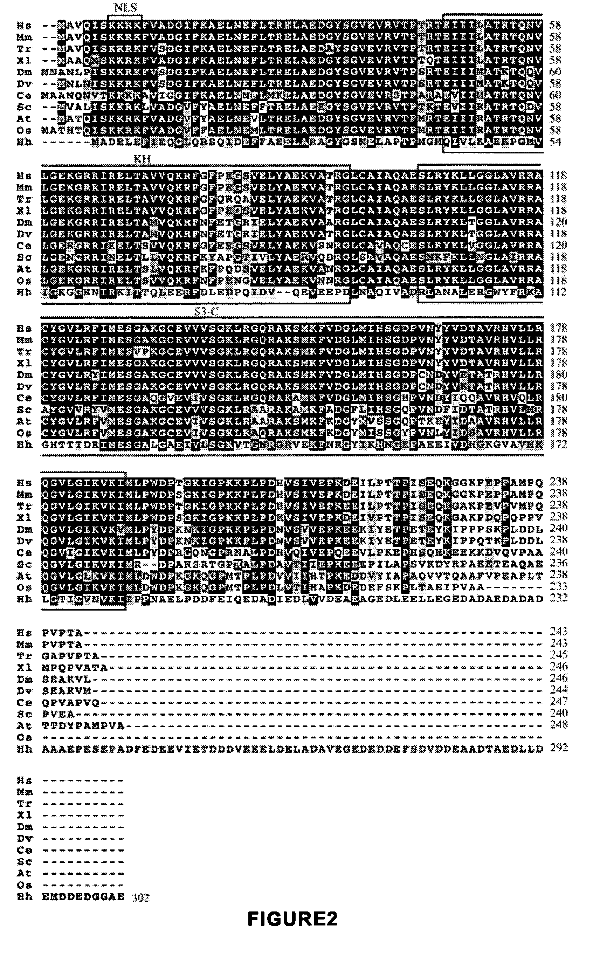

An S3a may easily be identified by aligning a query sequence (preferably a protein sequence) with known S3a protein sequences (see for example the alignments shown in Figures 1 and 2). The query sequence may be aligned (with known S3a sequences) using, for example, the VNTI AlignX multiple alignment program (InforMax, Bethesda, MD), with default settings for gap opening penalty of 10 and a gap extension of 0.05. Since S3a sequences are highly conserved, a person skilled in the art would readily be able to identify other S3a sequences by comparing any conserved regions of the query sequence against those of the known S3a sequences. These regions of high conservation are illustrated in Figure 2 by the three boxed regions. Therefore, a query sequence having corresponding regions of conservation would be identifiable as an S3a. S3as have also been shown to bind to eukaryotic initiation factors elF-2 and elF-3 (Westermann et al. (FEBS Letters Vol. 97, No. 1 (January 1979)).

The S3A-encoding nucleic acid sequences and the S3A protein sequences useful in the methods of the present invention encode or reflate to a 405 small subunit ribosomal protein. Any refernce herein to the term "S3A" therefore refers to a 40S small subunit ribosomal protein.

The term "S3a" as defined herein is taken to mean a protein, which when used in an alignment, such as the ones shown in Figures 1 or 2, aligns with known S3a protein sequences and comprises regions of conservation corresponding to the boxed regions shown in the alignment of Figure 2. Reference herein to an S3a-encoding nucleic acid is to a nucleic acid encoding a protein, which when used in an alignment, such as the one shown in Figures 1 or 2. aligns with known S3a protein sequences and comprises regions of conservation corresponding to the boxed regions shown in the alignment of Figure 2. S3as and S3a-encoding nucleic acids as defined above are useful in the methods of the invention.

The nucleic acid to be introduced into a plant may be any S3a-encoding sequence as hereinbefore defined. Preferably the nucleic acid is as represented by SEQ ID NO: 1. The nucleic acid to be introduced into a plant is preferably operably linked to a constitutive promoter for overexpression in a plant. The constitutive promoter is preferably a GOS2 promoter, further preferably a GOS2 promoter from rice. It should be clear that the applicability of the present invention is not restricted to the S3a represented by SEQ ID NO: 1, nor is the applicability of the invention restricted to expression of an S3a gene/nucleic acid when driven by a GOS2 promoter.

According to a preferred aspect of the present invention, enhanced or increased expression of the S3a nucleic acid is envisaged. Methods for obtaining enhanced or increased expression of genes or gene products are well documented in the art and include, for example, overexpression driven by a strong promoter, the use of transcription enhancers or translation enhancers.

The nucleic acid encoding an S3a may be derived from any source. The nucleic acid/gene encoding an S3a may be isolated from a microbial source, such as bacteria, yeast or fungi, or from a plant, algae or animal (including human) source. This nucleic acid may be modified from its native form in composition and/or genomic environment through deliberate human manipulation. The nucleic acid is preferably a homologous nucleic acid, i.e. a nucleic acid obtained from a plant, whether from the same plant species in which it is to be introduced or whether from a different plant species. The nucleic acid may be isolated from a dicotyledonous species, preferably from the family *Brassicaceae,* further preferably from *Arabidopsis thaliana.* More preferably, the S3a isolated from *Arabidopsis thaliana* is represented by SEQ ID NO: 1 and the amino acid sequence as represented by SEQ ID NO: 2.

The sequence represented by SEQ ID NO: 1 depicts an S3a from *Arabidopsis thaliana,* with SEQ ID NO: 2 being the corresponding amino acid sequence. Advantageously, the applicability of the present invention is not restricted to use of an S3a from *Arabidopsis thaliana,* as represented by SEQ ID NO: 1. The methods according to the present invention may also be practised using any S3a or S3a-encoding sequence as defined hereinabove.

The S3a or S3a-encoding nucleic acids useful in practising the method according to the invention may be:
(i) A sequence hybridising under stringent conditions to a 408 small subunit ribosomal protein -encoding nucleic acid;
(ii) An alternative splice variant of a 40S small subunit ribosomal, protein-encoding nucleic acid;
(iii) An allelic variant of a 40S small subunit ribosomal protein-encoding nucleic acid; and
(iv) A nucleic acid encoding an orthologue having In increasing order of preference at least 80%, 85%, 90%, 95% or more amino acid sequence identity to a 40S small subunit ribosomal protein

Also useful in performing methods of the invention are nucleic acids hybridising under stringent conditions with an S3a-encoding nucleic acid, preferably nucleic acids hybridising under"-stringent conditions to an S3a-encoding nucleic acid sequence as represented by SEQ ID NO: 1. Such hybridising sequences are those encoding S3a proteins that align with known S3a protein sequences and that comprise regions of conservation corresponding to the boxed regions shown in the alignment of Figure 2

The term "hybridisation" as defined herein is a process wherein substantially homologous complementary nucleotide sequences anneal to each other. The hybridisation process can occur entirely in solution, i.e. both complementary nucleic acids are in solution. Tools in molecular biology relying on such a process include the polymerase chain reaction (PCR; and all methods based thereon), subtractive hybridisation, random primer extension, nuclease S1 mapping, primer extension, reverse transcription, cDNA synthesis, differential display of RNAs, and DNA sequence determination. The hybridisation process can also occur with one of the complementary nucleic acids immobilised to a matrix such as magnetic beads, Sepharose beads or any other resin. Tools in molecular biology relying on such a process include the isolation of poly (A*) RNA. The hybridisation process can furthermore occur with one of the complementary nucleic acids immobilised to a solid support such as a nitro-cellulose or nylon membrane or immobilised by e.g. photolithography to, for example, a siliceous glass support (the latter known as nucleic acid arrays or microarrays or as nucleic acid chips). Tools in molecular biology relying on such a process include RNA and DNA gel blot analysis, colony hybridisation, plaque hybridisation, *in situ* hybridisation and microarray hybridisation. In order to allow hybridisation to occur, the nucleic acid molecules are generally thermally or chemically denatured to melt a double strand into two single strands and/or to remove hairpins or other secondary structures from single stranded nucleic acids. The stringency of hybridisation is influenced by conditions such as temperature, salt concentration and hybridisation buffer composition. Hybridisation preferably occurs under stringent conditions. Stringent conditions are those that (1) employ low ionic strength and high temperature for washing, for example, 0.5M sodium phosphate buffer pH 7.2, 1mM EDTA pH 8.0 in 7% SDS at either 65°C or 55°C, or (2) employ during hybridization a denaturing agent such as formamide, for example, 50% (vol/vol) formamide with 0.1% bovine serum albumin, 0.1% Ficoll, 0.1% polyvinylpolypyrrolidone, 0.05 M sodium phosphate buffer at pH 6-5 with 0.75 M NaCl, 0.075 M sodium citrate at 42°C. A specific example includes the use of 50% formamide, 5XSSC (0.75 M NaCl, 0.075 M sodium citrate), 50 mM sodium phosphate (pH 6.8), 0.1% sodium pyrophosphate, 5X Denhard's solution, sonicated salmon sperm DNA (50nm/ml), 0.1% SDS and 10% dextran sulfate at 55°C, with washes at 55°C in 0.2XSSC and 0.1 % SDS. A skilled person can readily determine and vary the stringency conditions appropriately to obtain a clear and detectable hybridization signal.

Therefore according to the invention, there is provided, a method for increasing seed yield in plants, comprising transforming a plant with a gene contruct comprising a nucleic acid sequence capable of hybridising under stringent conditions to an S3a-encoding nucleic acid sequence, preferably to an S3a-encoding nucleic acid as represented by SEQ ID NO: 1.

Also useful in performing the methods of the invention are alternative splice variants of an S3a-encoding nucleic acid, preferably an alternative splice variant of an S3a-encoding nucleic acid as represented by the sequence of SEQ ID NO: 1. Splice variants suitable for use in performing the methods of the invention are those encoding S3a proteins that align with known S3a protein sequences and that comprise regions of conservation corresponding to the boxed regions shown in the alignment of Figure 2. The term "alternative splice variant" as used herein encompasses variants of a nucleic acid sequence in which selected introns and/or exons have been excised, replaced or added. Such variants will be ones in which the biological activity of the protein remains unaffected, which can be achieved by selectively retaining functional segments of the protein. Such splice variants may be found in nature or may be manmade. Methods for making such splice variants are well known in the art.

Therefore, the invention also provides a method for increasing seed yield in plants, comprising transforming a plant with a gene construct comprising an alternative splice variant of a 40S subunit ribosomal protein (S3a)-encoding nucleic acid, preferably an alternative splice variant of an S3a-encoding nucleic acid as represented by SEQ ID NO: 1.

Also useful in performing the methods of the invention are allelic variants of an S3a-encoding nucleic acid, preferably an allelic variant of an S3a-encoding nucleic acid as represented by SEQ ID NO: 1. Allelic variants suitable for use in performing the methods of the invention are those encoding S3a proteins that align with known S3a protein sequences and that comprise regions of conservation corresponding to the boxed regions shown in the alignment of Figure 2.

Allelic variants exist in nature and encompassed within the methods of the present invention is the use of these natural alleles. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

Therefore, the invention also provides a method for increasing seed yield in plants, comprising / transforming a plant with a gene construct comprising an allelic variant of a 40S small subunit ribosomal protein (S3a)-encoding nucleic acid as represented by SEQ ID NO: 1.

Further advantageously, the methods according to the present invention may also be practised using orthologues of an S3a, such as an S3a represented by SEQ ID NO: 2. Nucleic acids encoding homologues or derivatives of S3as may readily be determined using routine techniques well known to persons skilled in the art.

Orthologues in, for example, monocot plant species may easily be found by performing a so-called reciprocal blast search. This may be done by a first blast involving blasting the sequence in question (for example, SEQ ID NO: 1 or SEQ ID NO: 2) against any sequence database, such as the publicly available NCBI database which may be found at http://www.ncbi.nlm.nih.gov. If orthologues in rice were sought, the sequence in question would be blasted against, for example, the 28,469 full-length cDNA clones from *Oryza sativa* Nipponbare available at NCBI. BLASTn may be used when starting from nucleotides or TBLASTX when starting from the protein, with standard default values (expectation 10, alignment 50). The blast results may be filtered. The full-length sequences of either the filtered results or the non-filtered results are then blasted back (second blast) against the sequence in question (SEQ ID NO: 1 or 2). The results of the first and second blasts are then compared. In the case of large families, ClustalW is used followed by a neighbour joining tree to help visualize the clustering.

Therefore, the invention also provides a method for increasing seed yield in plants, comprising transforming a plant with a gene construct comprising a nucleic acid encoding an orthologue having in increasing order of preference at least 80%, 85%, 90%, 95% or more amino acid sequence identity to a 40S small subunit ribosomal protein. Preferably, the orthologue has in increasing order of preference at least 55%, 60%, 65%, 70%, 75%, 80%, 85%, 90%, 95% sequence identity to an amino acid sequence as represented by SEQ ID NO: 2 and which orthologue aligns with known S3a protein sequences and comprises regions of conservation corresponding to the boxed regions shown in the alignment of Figure 2.

The invention also provides genetic constructs and vectors to facilitate introduction and/or expression of the nucleotide sequences useful in the methods according to the invention.

Therefore, there is provided a gene construct comprising:
(i) An S3a-encoding nucleic acid, preferably as represented by SEQ ID NO: 1;
(ii) one or more control sequences capable of driving expression of the nucleic acid sequence of (i); and optionally
(iii) a transcription termination sequence.

Constructs useful in the methods according to the present invention may be constructed using recombinant DNA technology well known to persons skilled in the art. The gene constructs may be inserted into vectors, which may be commercially available, suitable for transforming into plants and suitable for expression of the gene of interest in the transformed cells.

Plants are transformed with a gene construct comprising the sequence of interest (i.e., an S3a-encoding nucleic acid as defined hereinabove. The sequence of interest is operably linked to one or more control sequences (at least to a promoter). The terms "regulatory element", "control sequence" and "promoter" are all used interchangeably herein and are to be taken in a broad context to refer to regulatory nucleic acid sequences capable of effecting expression of the sequences to which they are ligated. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative which confers, activates or enhances expression of a nucleic acid molecule in a cell, tissue or organ. The term "operably linked" as used herein refers to a functional linkage between the promoter sequence and the gene of interest, such that the promoter sequence is able to initiate transcription of the gene of interest.

Advantageously, any type of promoter may be used to drive expression of the S3a-encoding nucleic acid.

Preferably, the nucleic acid encoding an S3a is operably linked to a constitutive promoter. The term "constitutive" as defined herein refers to a promoter that is expressed predominantly in more than one tissue or organ and predominantly at any stage in the life cycle of a plant. Preferably, the constitutive promoter is expressed predominantly throughout the plant. Preferably, the constitutive promoter is the GOS2 promoter from rice.

Examples of other constitutive promoters suitable for use in the methods of the invention are listed in Table A below.

**Table A: Examples of constitutive promoters for use in performance of the invention**

| **Gene source** | **Expression pattern** | **Reference** |
|---|---|---|
| Actin | constitutive | McElroy et al, Plant Cell, 2: 163-171, 1990 |
| CAMV 35S | constitutive | Odell et al, Nature, 313: 810-812, 1985 |
| CaMV 19S | constitutive | Nilsson et al., Physiol. Plant. 100:456-462,1997 |
| GOS2 | constitutive | de Pater et al, Plant J Nov;2(6):837-44, 1992 |
| ubiquitin | constitutive | Christensen et al, Plant Mol. Biol. 18: 675-689, 1992 |
| rice cyclophilin | constitutive | Buchholz et al, Plant Mol Biol. 25(5): 837-43, 1994 ' |
| maize H3 histone | constitutive | Lepetit et al, Mol. Gen. Genet. 231:276-285, 1992 |
| actin 2 | constitutive | An et al, Plant J. 10(1); 107-121, 1996 |

Optionally, one or more terminator sequences may also be used in the construct introduced into a plant. The term "terminator" encompasses a control sequence which is a DNA sequence at the end of a transcriptional unit which signals 3' processing and polyadenylation of a primary transcript and termination of transcription. Additional regulatory elements may include transcriptional as well as translational enhancers. Those skilled in the art will be aware of terminator and enhancer sequences which may be suitable for use in performing the invention. Such sequences would be known or may readily be obtained by a person skilled in the art.

The genetic constructs of the invention may further include an origin of replication sequence which is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

The genetic construct may optionally comprise a selectable marker gene. As used herein, the term "selectable marker gene" includes any gene which confers a phenotype on a cell in which it is expressed to facilitate the identification and/or selection of cells which are transfected or transformed with a nucleic acid construct of the invention. Suitable markers may be selected from markers that confer antibiotic or herbicide resistance, that introduce a new metabolic trait or that allow visual selection. Examples of selectable marker genes include genes conferring resistance to antibiotics (such as nptII that phosphorylates neomycin and kanamycin, or hpt, phosphorylating hygromycin), to herbicides (for example bar which provides resistance to Basta; aroA or gox providing resistance against glyphosate), or genes that provide a metabolic trait (such as manA that allows plants to use mannose as sole carbon source). Visual marker genes result in the formation of colour (for example β-glucuronidase, GUS), luminescence (such as luciferase) or fluorescence (Green Fluorescent Protein, GFP, and derivatives thereof).

The present invention also encompasses plants obtainable by the methods according to the present invention- The present invention therefore provides plants obtainable by the method according to the present invention, which plants have introduced and expressed therein a nucleic acid encoding an S3a protein, as defined hereinabove.

The invention also provides a method for the production of transgenic plants having increased seed yield, comprising introduction and expression in a plant of an S3a-encoding nucleic acid, preferably as represented by SEQ ID NO: 1.

More specifically, the present invention provides a method for the production of transgenic plants having increased seed yield, which method comprises:
(i) transforming a plant with a gene construct comprising a 40S small subunit ribosomal protein (S3a)-encoding nucleic acid, preferably as represented by SEQ ID NO: 1;
(ii) cultivating the plant cell under conditions promoting regeneration and mature plant growth.

The nucleic acid may be introduced directly into a plant cell or into the plant itself (including introduction into a tissue, organ or any other part of a plant). According to a preferred feature of the present invention, the nucleic acid is preferably introduced into a plant by transformation.

The term "transformation" as referred to herein encompasses the transfer of an exogenous polynucleotide into a host cell, irrespective of the method used for transfer. Plant tissue capable of subsequent clonal propagation, whether by organogenesis or embryogenesis may be transformed with a genetic construct of the present invention and a whole plant regenerated therefrom. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

Transformation of plant species is now a fairly routine technique. Advantageously, any of several transformation methods may be used to introduce the gene of interest into a suitable ancestor cell. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., 1882, Nature 296, 72-74; Negrutiu I. et al., June 1987, Plant Mol. Biol. 8, 363-373); electroporation of protoplasts (Shillito R.D. et al., 1985 Bio/Technol 3, 1099-1102); microinjection into plant material (Crossway A. et al., 1986, Mol, Gen Genet 202, 179-185); DNA or RNA-coated particle bombardment (Klein T.M. et al., 1987, Nature 327, 70) infection with (non-integrative) viruses and the like. Transgenic rice plants expressing an S3a are preferably produced via *Agrobacterium*-mediated transformation using any of the well known methods for rice transformation, such as described in any of the following: published European patent application EP 1198985 A1, Aldemita and Hodges (Planta, 199, 612-617, 1996); Chan et al. (Plant Mol. Biol. 22 (3) 491-506, 1993), Hiei et al. (Plant J. 6 (2) 271-282, 1994), which disclosures are incorporated by reference herein as if fully set forth. In the case of com transformation, the preferred method is as described in either Ishida et al. (Nat. Biotechnol. 1996 Jun; 14(6): 745-50) or Frame et al. (Plant Physiol. 2002 May; 129(1): 13-22), which disclosures are incorporated by reference herein as if fully set forth.

Generally after transformation, plant cells or cell groupings are selected for the presence of one or more markers which are encoded by plant-expressible genes co-transferred with the gene of interest, following which the transformed material is regenerated into a whole plant.

Following DNA transfer and regeneration, putatively transformed plants may be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed to give homozygous second generation (or T2) transformants, and the T2 plants further propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced in the parent by the methods according to the invention. The invention also includes host cells containing an isolated S3a-encoding nucleic acid. Preferred host cells according to the invention are plant cells. The invention also extends to harvestable parts of a plant such as but not limited to seeds, leaves, fruits, flowers, stem cultures, rhizomes, tubers and bulbs.

Homologous recombination allows introduction in a genome of a selected nucleic acid at a defined selected position. Homologous recombination is a standard technology used routinely in biological sciences for lower organisms such as yeast or the moss physcomitrella. Methods for performing homologous recombination in plants have been described not only for model plants (Offringa et al. Extrachromosomal homologous recombination and gene targeting in plant cells after Agrobacterium-mediated transformation. 1990 EMBO J. 1990 Oct; 9(10):3077-84) but also for crop plants, for example rice (Terada R, Urawa H, Inagaki Y, Tsugane K, Iida S. Efficient gene targeting by homologous recombination in rice. Nat Biotechnol. 2002. lida and Terada: A tale of two integrations, transgene and T-DNA: gene targeting by homologous recombination in rice. Curr Opin Biotechnol. 2004 Apr; 15(2):132-8). The nucleic acid to be targeted (which may be an SSa-encoding nucleic acid as hereinbefore defined) need not be targeted to the locus of an S3a -encoding gene, but may be introduced in, for example, regions of high expression. The nucleic acid to be targeted may be an improved allele used to replace the endogenous gene or may be introduced in addition to the endogenous gene.

The present invention also encompasses the use of nucleic acids encoding S3as and the use of S3a polypeptides.

One such use of course relates to the use of an S3a as defined hereinabove in improving seed yield in plants. The seed yield may include one or more of the following: increased number of (filled) seeds, increased seed weight, increased harvest index and increased TKW, among others. The S3a-encoding nucleic acid may be as represented by SEQ ID NO: 1 and the S3a protein may be an amino acid as represented by SEQ ID NO: 2.

Nucleic acids encoding S3as, and S3a polypeptides, as hereinbefore defined may also find use in breeding programmes. The S3a-encoding nucleic acid may be as represented by SEQ ID NO: 1; or the S3a may be an amino acid as represented by SEQ ID NO: 2. For example, the S3a-encoding nucleic acid or a part thereof may be on a chromosome (or a part thereof), preferably together with one or more related family members. In an example of such a breeding programme, a DNA marker is identified which may be genetically linked to a gene capable of modulating expression of a nucleic acid encoding an S3a protein in a plant, which gene may be a gene encoding the S3a protein itself or any other gene which may directly or indirectly influence expression of a gene encoding an S3a protein and/or activity of the S3a protein itself. This DNA marker may then used in breeding programs to select plants having improved growth characteristics.

Allelic variants of an S3a may also be used in conventional breeding programmes, such as in marker-assisted breeding. Such breeding programmes sometimes require the introduction of allelic variations in the plants by mutagenic treatment of a plant. One suitable mutagenic method is EMS mutagenesis. Identification of allelic variants then takes place by, for example, PCR. This is followed by a selection step for selection of superior allelic variants of the sequence in question and which give improved growth characteristics in a plant. Selection is typically carried out by monitoring growth performance of plants containing different allelic variants of the sequence in question, for example, different allelic variants of SEQ ID NO: 1. Monitoring growth performance can be done in a greenhouse or in the field. Further optional steps include crossing plants, in which the superior allelic variant was identified, with another plant. This could be used, for example, to make a combination of interesting phenotypic features.

An S3a-encoding nucleic acid may also be used as probes for genetically and physically mapping the genes that they are a part of, and as markers for traits linked to those genes. Such information may be useful in plant breeding in order to develop lines with desired phenotypes. Such use of an S3a-encoding nucleic acid requires only a nucleic acid sequence of at least 15 nucleotides in length. The S3a-encoding nucleic acid may be used as restriction fragment length polymorphism (RFLP) markers. Southern blots (Maniatis) of restriction-digested plant genomic DNA may be probed with the S3a-encoding nucleic acid. The resulting banding patterns may then be subjected to genetic analyses using computer programs such as MapMaker (Lander et al. (1987) Genomics 1:174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the S3a-encoding nucleic acid in the genetic map previously obtained using this population (Botstein et al. (1980) Am, J. Hum. Genet. 32:314-331).

The production and use of plant gene-derived probes for use in genetic mapping is described in Bematzky and Tanksley (1986) Plant Mol. Biol. Reporter 4:37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping.
Such methodologies are well known to those skilled in the art.

The nucleic acid probes may also be used for physical mapping (i.e., placement of sequences on physical maps; see Hoheisel et al. In, Non-mammalian Genomic Analysis: A Practical Guide, Academic press 1996, pp. 319-346, and references cited therein).

In another embodiment, the nucleic acid probes may be used in direct fluorescence *in situ* hybridization (FISH) mapping (Trask (1991) Trends Genet. 7:149-154). Although current methods of FISH mapping favor use of large clones (several to several hundred KB; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

Nucleic acids encoding S3as and S3a polypeptides may also find use as growth regulators. The S3a-encoding nucleic acid may be a nucleic acid as represented by SEQ ID NO: 1 and the S3a protein/polypeptide may be an amino acid as represented by SEQ ID NO: 2. Since these S3as are useful in improving seed yield in plants, the S3as would also be useful growth regulators, such as herbicides or growth stimulators.

The methods according to the present invention result in plants having increased seed yield, as described hereinbefore. These improved seed yield characteristics may also be combined with other economically advantageous traits, such as further yield-enhancing traits, tolerance to various stresses, traits modifying various architectural features and/or biochemical and/or physiological features.

### Description of figures

The present invention will now be described with reference to the following figures in which:
**Fig. 1** shows an alignment of S3a protein sequences made using the VNTI AlignX multiple alignment program (InforMax, Bethesda, MD), with default settings for gap opening penalty of 10 and a gap extension of 0.05. Residues appearing in white against a black background are identical; residues appearing in white against a gray background are either conservative or block of similar residues, as defined by VNTI options.
**Fig. 2** shows an alignment taken from Lyamouri et al. (Gene 294 (2002) 147-156) which shows the high degree of conservation in S3a proteins from various species: *H. sapiens* (Hs), *M*. *musculus* (Mm), *T rubripes* (Tr), *X. laevis* (XI), *D. melanogaster* (Dm), *D. virilis* (Dv), *C. elegans* (Ce), *S. cerovisiae* (Sc), *A. thaliana* (At), *O. sativa* (Os) and *H. holobium* (Hh). Black shading represents identical amino acids, while light grey represents conserved amino acid substitutions. Gaps are indicated by dashes. Regions of highest conservation are boxed.

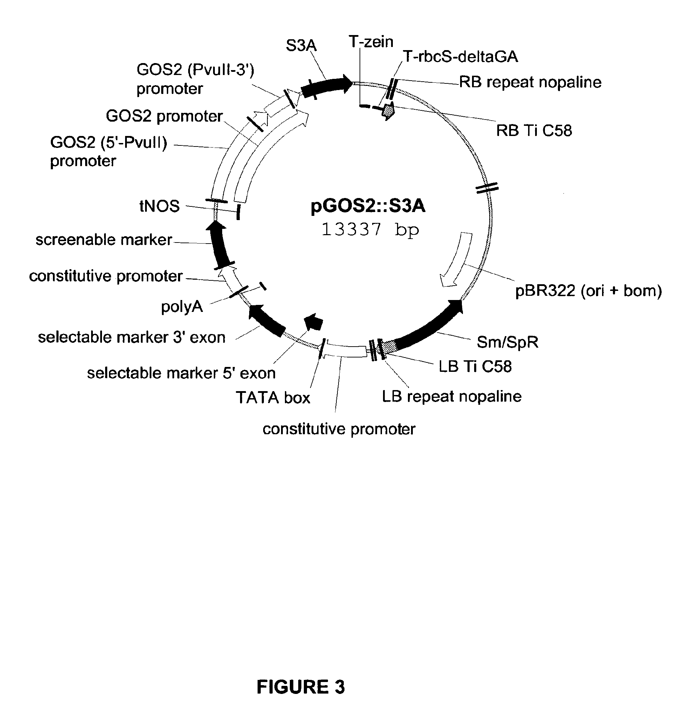

**Fig. 3** shows a binary vector for expression in *Oryza sativa* of the *Arabidopsis thaliana* cyc07putative S-phase-specific 40S S3a ribosomal protein gene (internal reference CDS0730) under the control of the rice GOS2 promoter (internal reference PRO0129).

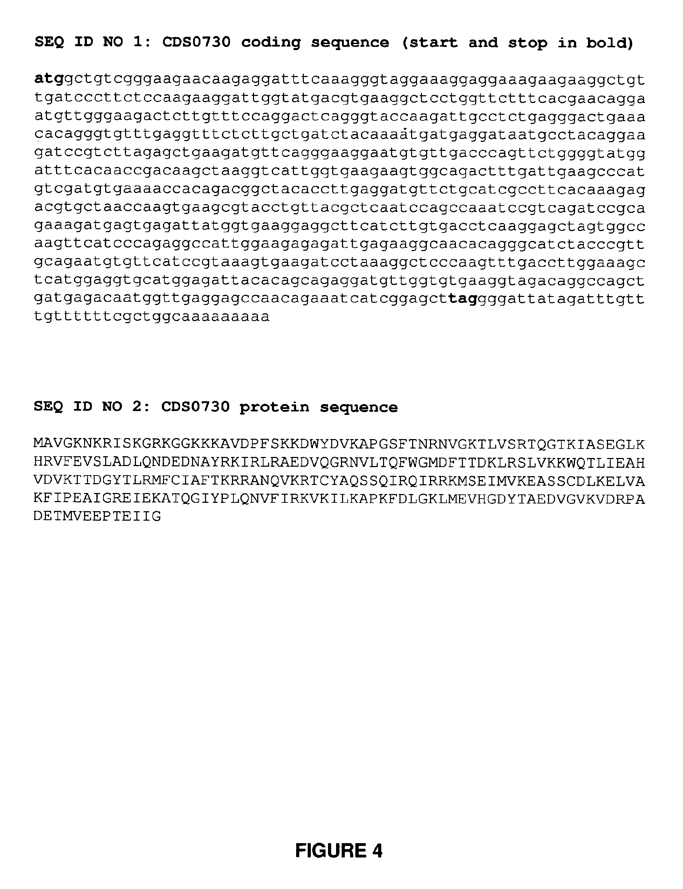

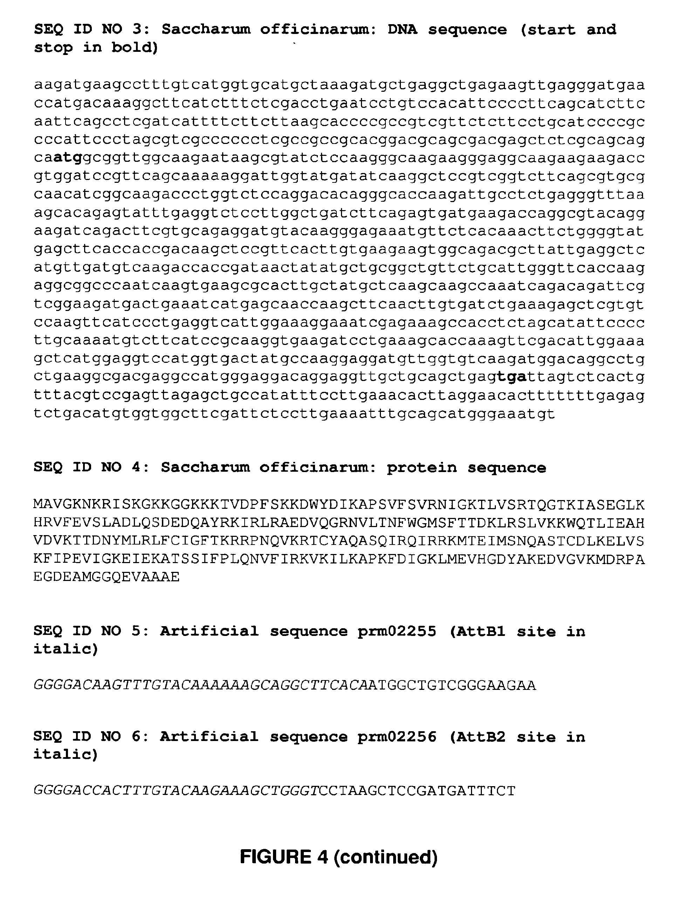

**Fig. 4** details examples of sequences useful in performing the methods according to the present invention.

### Examples

The present invention will now be described with reference to the following examples, which are by way of illustration alone.

DNA manipulation, unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfase (1993) by R.D.D. Croy, published by BIOS Scientific Publications Ltd (UK) and Blackwell Scientific Publications (UK).

### Example 1: Gene Cloning

The *Arabidopsis* S3a (S-phase specific 40S cyc07 was amplified by PCR using as template an *Arabidopsis thaliana* seedling cDNA library (Invitrogen, Paisley, UK). After reverse transcription of RNA extracted from seedlings, the cDNAs were cloned into pCMV Sport 6.0. Average insert size of the bank was 1.5 kb, and original number of clones was of 1.59x1 07 cfu. The original titer was determined to be 9.6x10⁸ cfu/ml, and became after a first amplification 6x10¹¹ cfu/ml. After plasmid extraction, 200 ng of template was used in a 50 µl PCR mix, Primers prm02255 (sense, start codon in bold, AttB1 site in italic: 5' *GGGGACAAGTTTGTACAAAAAAGCAGGCTTCACA*ATGGCTGTCGGGAAGAA 3') and prm02256 (reverse, complementary, stop codon in bold, AttB2 site in italic: 5' *GGGGACCACTTTGTACAAGAAAGCTGGGTCC*TAAGCTCCGATGATTTCT 3'), which include the AttB sites for Gateway recombination, were used for PCR amplification. PCR was performed using Hifi Taq DNA polymerase under standard conditions. A PCR fragment of 789 bp was amplified and purified also using standard methods. The first step of the Gateway procedure, the BP reaction, was then performed, during which the PCR fragment recombines *in vivo* with the pDONR201 plasmid to produce, according to the Gateway terminology, an "entry clone", p2782. Plasmid pDONR201 was purchased from Invitrogen, as part of the Gateway^{®} technology.

### Example 2: Vector Construction

The entry clone p2782 was subsequently used in an LR reaction with p0640, a destination vector used for *Oryza sativa* transformation. This vector contains as functional elements within the T-DNA borders: a plant selectable marker; a plant screenable marker; and a Gateway cassette intended for LR *in vivo* recombination with the sequence of interest already cloned in the entry clone. A rice GOS2 promoter for constitutive expression (PRO0129 is located upstream of this Gateway cassette. After the LR recombination step, the resulting expression vector as shown in Figure 3 was transformed into *Agrobacterium* and subsequently into *Oryza sativa* plants. Transformed rice plants were allowed to grow and were then examined for the parameters described in Example 3.

### Example 3: Evaluation and Results

Approximately 15 to 20 independent T0 rice transformants were generated. The primary transformants were transferred from tissue culture chambers to a greenhouse for growing and harvest of T1 seed. 6 events, of which the T1 progeny segregated 3:1 for presence/absence of the transgene, were retained. For each of these events, approximately 10 T1 seedlings containing the transgene (hetero- and homo-zygotes), and approximately 10 T1 seedlings lacking the transgene (nullizygotes), were selected by monitoring visual marker expression. Some T1 events were further evaluated in the T2 generation following the same evaluation procedure as for the T1 generation.

### Statistical analysis: F-test

A two factor ANOVA (analysis of variants) was used as a statistical model for the overall evaluation of plant phenotypic characteristics. An F-test was carried out on all the parameters measured of all the plants of all the events transformed with the gene of the present invention. The F-test was carried out to check for an effect of the gene over all the transformation events and to verify for an overall effect of the gene, also known as a global gene effect. The threshold for significance for a true global gene effect was set at a 5% probability level for the F-test. A significant F-test value points to a gene effect, meaning that it is not only the presence or position of the gene that is causing the differences in phenotype.

### 3.1 Seed-related parameter measurements

The mature primary panicles were harvested, bagged, barcode-labelled and then dried for three days in the oven at 37°C. The panicles were then threshed and all the seeds were collected and counted. The filled husks were separated from the empty ones using an airblowing device. The empty husks were discarded and the remaining fraction was counted again. The filled husks were weighed on an analytical balance. This procedure resulted in the set of seed-related parameters described below.

The Table of results below show the p values from the F test for the T1 evaluations, the T2 evaluations and the combined p values from the F tests for the T1 and T2 evaluations. A combined analysis may be considered when two experiments have been carried out on the same events. This may be useful to check for consistency of the effects over the two experiments and to increase confidence in the conclusion. The method used is a mixed-model approach that takes into account the multilevel structure of the data (i.e. experiment - event - segregants). P-values are obtained by comparing likelihood ratio test to chi square distributions. Each of the tables also gives the % difference between the transgenics and the corresponding nullizygotes for each generation.

### 3.1.1 Number of filled seeds

The number of filled seeds was determined by counting the number of filled husks that remained after the separation step. As shown in Table 1 below, the p value from the F test for the T1 and T2 evaluation combined was significant (with a p value of 0.0071) indicating that the presence of the construct in the plants has a significant effect on the number of filled seeds of transgenic plants.

**Table 1**

| **Number of filled seeds** | | |
|---|---|---|
| | % Difference | P value |
| T1 | 22 | 0.354 |
| T2 | 15 | 0.1829 |
| Combined | | 0.0071 |

### 3.1.2 Total seed yield per plant

The total seed yield was measured by weighing all filled husks harvested from a plant. As shown in Table 1 below, the p value from the F test for the T1 and T2 evaluation combined was significant (with a p value of 0.0016) indicating that the presence of the construct in the plants has a significant effect on the total seed weight of transgenic plants.

**Table 2**

| **Total Seed Weight** | | |
|---|---|---|
| | % Difference | P value |
| T1 | 29 | 0.0266 |
| T2 | 20 | 0.1037 |
| Combined | | 0.0016 |

### 3.1.3 Harvest index of plants

The harvest index in the present invention is defined as the ratio between the total seed yield and the above ground area (mm²), multiplied by a factor 10⁶. As shown in Table 3 below, the p value from the F test for the T1 and T2 evaluation combined (and individually) was significant (with a p value of 0.0005) indicating that the presence of the construct in the plants has a significant effect on the harvest index of transgenic plants.

**Table 3**

| **Harvest index** | | |
|---|---|---|
| | % Difference | P value |
| T1 | 15 | 0.0358 |
| T2 | 14 | 0.0365 |
| Combined | | 0.0005 |

### 3.1.4 Thousand Kernel Weight (TKW)

This parameter is extrapolated from the number of filled seeds counted, and their total weight. As shown in Table 4 below, the p value from the F test for the T1 and T2 evaluation combined was significant (with a p value of 0.0405) indicating that the presence of the construct in the plants has a significant effect on the TKW of transgenic plants.

**Table 4**

| **Thousand Kernel Weight** | | |
|---|---|---|
| | **% Difference** | **P value** |
| T1 | 3 | 0.3353 |
| T2 | 2 | 0.1257 |
| Combined | | 0.0405 |

## Claims

1. Method for increasing seed yield in plants relative to corresponding wild type plants, comprising transforming a plant with a gene construct comprising a nucleic acid encoding a 40S small subunit ribosomal protein.

2. Method according to claim 1, wherein said nucleic acid is:
(i) A sequence hybridising under stringent conditions to a 40S small subunit ribosomal protein -encoding nucleic acid;
(ii) An alternative splice variant of a 40S small subunit ribosomal protein-encoding nucleic acid;
(iii) An allelic variant of a 40S small subunit ribosomal protein-encoding nucleic acid; and
(iv) A nucleic acid encoding an orthologue having in increasing order of preference at least 80%, 85%, 90%. 95% or more amino acid sequence identity to a 40S small subunit ribosomal protein.

3. Method according to claim 1, wherein said 40S small subunit ribosomal protein is as represented by SEQ ID NO: 2.

4. Method according to claim 1, wherein said increased seed yield is selected from one or more of: (i) increased seed biomass; (ii) increased number of (filled) seeds; (iii) increased seed size; (iv) increased seed volume; (v) increased harvest index; or (vi) increased thousand kernel weight (TKW).

5. Method according to any one of claims 1 to 4, wherein said nucleic acid encoding a 40S small subunit ribosomal protein, is derived from a plant, preferably from a dicotyledonous plant, further preferably from the family *Brassicaceae,* more preferably the nucleic acid is from *Arabidopsis thaliana.*

6. Method according to any one of claims 1 to 5, wherein said nucleic acid sequence encoding a 40S small subunit ribosomal protein, is overexpressed in a plant.

7. Method according to any one of claims 1 to 6, wherein expression of said nucleic acid encoding a 40S small subunit ribosomal protein, is driven by a constitutive promoter, particularly the GOS2 promoter.

8. Method for the production of a transgenic plant having increased seed yield relative to corresponding wild type plants, which method comprises:
(i) transforming a plant with a gene construct comprising a 40S small subunit ribosomal protein-encoding nucleic acid as defined in claims 2 or 3;
(ii) cultivating the plant cell under conditions promoting regeneration and mature plant growth.

9. Use of a gene construct comprising a 40S small subunit ribosomal protein-encoding nucleic acid as defined in claims 2 or 3, in increasing seed yield of plants relative to corresponding wild type plants.

10. Use according to claim 9, wherein said seed yield includes one or more of the following: increased number of (filled) seeds, increased seed weight, increased harvest index and increased TKW.

11. Use according to claim 9 or 10, wherein said 40S small subunit ribosomal protein-encoding nucleic acid is as represented by SEQ ID NO: 1 and wherein said 40S small subunit ribosomal protein is an amino acid as represented by SEQ ID NO: 2.

## Patentansprüche

1. Verfahren zur Erhöhung der Samenausbeute in Pflanzen relativ zu entsprechenden Wildtyp-Pflanzen, umfassend das Transformieren einer Pflanze mit einem Genkonstrukt, das eine Nukleinsäure umfasst, die für ein Protein der kleinen ribosomalen 40S-Untereinheit codiert.

2. Verfahren nach Anspruch 1, wobei die Nukleinsäure Folgendes ist:
(i) eine Sequenz, die unter stringenten Bedingungen an eine Nukleinsäure hybridisiert, die für ein Protein der kleinen ribosomalen 40S-Untereinheit codiert;
(ii) eine alternative Spleißvariante einer Nukleinsäure, die für ein Protein der kleinen ribosomalen 40S-Untereinheit codiert;
(iii) eine allelische Variante einer Nukleinsäure, die für ein Protein der kleinen ribosomalen 40S-Untereinheit codiert; und
(iv) eine Nukleinsäure, die für ein Ortholog mit mindestens 80%, 85%, 90%, 95% oder mehr, wobei die Präferenz in dieser Reihenfolge zunimmt, Aminosäuresequenz-Identität zu einem Protein der kleinen ribosomalen 40S-Untereinheit codiert.

3. Verfahren nach Anspruch 1, wobei das Protein der kleinen ribosomalen 40S-Untereinheit durch die SEQ ID NR: 2 wiedergegeben wird.

4. Verfahren nach Anspruch 1, wobei die erhöhte Samenausbeute ausgewählt ist aus einem oder mehreren der Folgenden: (i) erhöhte Samen-Biomasse; (ii) erhöhte Anzahl (gefüllter) Samen; (iii) erhöhte Samengröße; (iv) erhöhtes Samenvolumen; (v) erhöhter Ernteindex oder (vi) erhöhtes Tausendkorngewicht (TKW).

5. Verfahren nach einem der Ansprüche 1 bis 4, wobei die Nukleinsäure, die für ein Protein der kleinen ribosomalen 40S-Untereinheit codiert, von einer Pflanze, vorzugsweise von einer Dikotyledone, weiterhin bevorzugt aus der Familie Brassicaceae, stammt und die Nukleinsäure stärker bevorzugt aus *Arabidopsis thaliana* ist.

6. Verfahren nach einem der Ansprüche 1 bis 5, wobei die Nukleinsäuresequenz, die für ein Protein der kleinen ribosomalen 40S-Untereinheit codiert, in einer Pflanze überexprimiert wird.

7. Verfahren nach einem der Ansprüche 1 bis 6, wobei die Expression der Nukleinsäure, die für ein Protein der kleinen ribosomalen 40S-Untereinheit codiert, durch einen konstitutiven Promotor, insbesondere den GOS2-Promotor, angetrieben wird.

8. Verfahren zur Herstellung einer transgenen Pflanze mit erhöhter Samenausbeute relativ zu entsprechenden Wildtyp-Pflanzen, wobei das Verfahren Folgendes umfasst:
(i) Transformieren einer Pflanze mit einem Genkonstrukt, das eine Nukleinsäure, die für ein Protein der kleinen ribosomalen 40S-Untereinheit codiert, nach den Ansprüchen 2 oder 3 umfasst;
(ii) Kultivieren der Pflanzenzelle unter Bedingungen, welche die Regeneration und das Wachstum reifer Pflanzen fördern.

9. Verwendung eines Genkonstrukts, das eine Nukleinsäure, die für ein Protein der kleinen ribosomalen 40S-Untereinheit codiert, nach den Ansprüchen 2 oder 3 umfasst, zur Erhöhung der Samenausbeute von Pflanzen relativ zu entsprechenden Wildtyp-Pflanzen.

10. Verwendung nach Anspruch 9, wobei die Samenausbeute eines oder mehrere der Folgenden umfasst: erhöhte Anzahl an (gefüllten) Samen, erhöhtes Samengewicht, erhöhter Ernetindex und erhöhtes TKW.

11. Verwendung nach Anspruch 9 oder 10, wobei die Nukleinsäure, die für ein Protein der kleinen ribosomalen 40S-Untereinheit codiert, durch SEQ ID NR: 1 wiedergegeben wird und das Protein der kleinen ribosomalen 40S-Untereinheit eine Aminosäure ist, die durch SEQ ID NR: 2 wiedergegeben wird.

## Revendications

1. Méthode d'augmentation du rendement en graines chez des plantes par rapport aux plantes de type sauvage correspondantes, comprenant la transformation d'une plante par un gène chimère comprenant un acide nucléique codant pour une protéine ribosomale de la petite sous-unité 40S.

2. Méthode selon la revendication 1, dans laquelle ledit acide nucléique est
(i) une séquence d'hybridant dans des conditions stringentes à un acide nucléique codant pour une protéine ribosomale de la petite sous-unité 40S ;
(ii) un variant d'épissage alternatif d'un acide nucléique codant pour une protéine ribosomale de la petite sous-unité 40S ;
(iii) un variant allélique d'un acide nucléique codant pour une protéine ribosomale de la petite sous-unité 40S ; et
(iv) un acide nucléique codant pour un orthologue ayant, en ordre croissant de préférence, au moins 80%, 85%, 90%, 95% ou plus d'identité de séquence d'acides aminés avec une protéine ribosomale de la petite sous-unité 40S.

3. Méthode selon la revendication 1, dans laquelle ladite protéine ribosomale de la petite sous-unité 40S est telle que représentée par la SEQ. ID n°2.

4. Méthode selon la revendication 1, dans laquelle ledit rendement accru en graines est choisi parmi un ou plusieurs parmi (i) une biomasse accrue en graines ; (ii) un nombre accru de graines (remplies) ; (iii) une taille accrue des graines ; (iv) un volume accru des graines ; (v) un indice de moisson accru ; ou (vi) un poids de mille grains accru.

5. Méthode selon l'une quelconque des revendications 1 à 4, dans laquelle ledit acide nucléique codant pour une protéine ribosomale de la petite sous-unité 40S est dérivé d'une plante, préférablement d'une plante dicotylédone, plus préférablement issue de la famille des Brassicacées, tout préférablement l'acide nucléique est issu d'*Arabidopsis thaliana.*

6. Méthode selon l'une quelconque des revendications 1 à 5, dans laquelle ladite séquence d'acide nucléique codant pour une protéine ribosomale de la petite sous-unité 40S est surexprimée dans une plante.

7. Méthode selon l'une quelconque des revendications 1 à 6, dans laquelle l'expression dudit acide nucléique codant pour une protéine ribosomale de la petite sous-unité 40S est régulée par un promoteur constitutif, en particulier le promoteur GOS2.

8. Méthode de production d'une plante transgénique ayant un rendement en graines accru par rapport à celui de plantes de type sauvage correspondantes, laquelle méthode comprend :
(i) la transformation d'une plante par un gène chimère comprenant un acide nucléique codant pour une protéine ribosomale de la petite sous-unité 40S, tel que défini selon les revendications 2 ou 3 ;
(ii) la culture de la cellule végétale dans des conditions favorisant la régénération et la croissance de la plante mature.

9. Utilisation d'un gène chimère comprenant un acide nucléique codant pour une protéine ribosomale de la petite sous-unité 40S, tel que défini selon les revendications 2 ou 3, pour augmenter le rendement en graines chez des plantes par rapport aux plantes de type sauvage correspondantes.

10. Utilisation selon la revendication 9, dans laquelle ledit rendement en graines comporte un ou plusieurs parmi ce qui suit : nombre accru de graines (remplies), poids accru des graines, indice de moisson accru, et poids de mille grains accru.

11. Utilisation selon les revendications 9 ou 10, dans laquelle ledit acide nucléique codant pour une protéine ribosomale de la petite sous-unité 40S est tel que représenté par la SEQ. ID n°1 et dans laquelle ladite protéine ribosomale de la petite sous-unité 40S est un acide aminé tel que représenté par la SEQ. ID n°2.


### SEQUENCE LISTING

<110> CropDesign N.V.
<120> Plants having improved growth characteristics and method for making the same
<130> CD-112-PCT
<150> EP 04101179.2
   <151> 2004-03-22
<150> US 60/556,847
   <151> 2004-03-28
<160> 6
<170> PatentIn version 3.3
<210> 1
   <211> 830
   <212> DNA
   <213> Arabidopsis thaliana
<400> 1
   atggctgtcg ggaagaacaa gaggatttca aagggtagga aaggaggaaa gaagaaggct      60
   gttgatccct tctccaagaa ggattggtat gacgtgaagg ctcctggttc tttcacgaac     120
   aggaatgttg ggaagactct tgtttccagg actcagggta ccaagattgc ctctgaggga     180
   ctgaaacaca gggtgtttga ggtttctctt gctgatctac aaaatgatga ggataatgcc     240
   tacaggaaga tccgtcttag agctgaagat gttcagggaa ggaatgtgtt gacccagttc     300
   tggggtatgg atttcacaac cgacaagcta aggtcattgg tgaagaagtg gcagactttg     360
   attgaagccc atgtcgatgt gaaaaccaca gacggctaca ccttgaggat gttctgcatc     420
   gccttcacaa agagacgtgc taaccaagtg aagcgtacct gttacgctca atccagccaa     480
   atccgtcaga tccgcagaaa gatgagtgag attatggtga aggaggcttc atcttgtgac     540
   ctcaaggagc tagtggccaa gttcatccca gaggccattg gaagagagat tgagaaggca     600
   acacagggca tctacccgtt gcagaatgtg ttcatccgta aagtgaagat cctaaaggct     660
   cccaagtttg accttggaaa gctcatggag gtgcatggag attacacagc agaggatgtt     720
   ggtgtgaagg tagacaggcc agctgatgag acaatggttg aggagccaac agaaatcatc     780
   ggagcttagg ggattataga tttgtttgtt ttttcgctgg caaaaaaaaa                830
<210> 2
   <211> 261
   <212> PRT
   <213> Arabidopsis thaliana
<400> 2
   Met Ala Val Gly Lys Asn Lys Arg Ile Ser Lys Gly Arg Lys Gly Gly
   1               5                   10                  15
   Lys Lys Lys Ala Val Asp Pro Phe Ser Lys Lys Asp Trp Tyr Asp Val
               20                  25                  30
   Lys Ala Pro Gly Ser Phe Thr Asn Arg Asn Val Gly Lys Thr Leu Val
           35                  40                  45
   Ser Arg Thr Gln Gly Thr Lys Ile Ala Ser Glu Gly Leu Lys His Arg
       50                  55                  60
   Val Phe Glu Val Ser Leu Ala Asp Leu Gln Asn Asp Glu Asp Asn Ala
   65                  70                  75                  80
   Tyr Arg Lys Ile Arg Leu Arg Ala Glu Asp Val Gln Gly Arg Asn Val
                   85                  90                  95
   Leu Thr Gln Phe Trp Gly Met Asp Phe Thr Thr Asp Lys Leu Arg Ser
               100                 105                 110
   Leu Val Lys Lys Trp Gln Thr Leu Ile Glu Ala His Val Asp Val Lys
           115                 120                 125
   Thr Thr Asp Gly Tyr Thr Leu Arg Met Phe Cys Ile Ala Phe Thr Lys
       130                 135                 140
   Arg Arg Ala Asn Gln Val Lys Arg Thr Cys Tyr Ala Gln Ser Ser Gln
   145                 150                 155                 160
   Ile Arg Gln Ile Arg Arg Lys Met Ser Glu Ile Met Val Lys Glu Ala
                   165                 170                 175
   Ser Ser Cys Asp Leu Lys Glu Leu Val Ala Lys Phe Ile Pro Glu Ala
               180                 185                 190
   Ile Gly Arg Glu Ile Glu Lys Ala Thr Gln Gly Ile Tyr Pro Leu Gln
           195                 200                 205
   Asn Val Phe Ile Arg Lys Val Lys Ile Leu Lys Ala Pro Lys Phe Asp
       210                 215                 220
   Leu Gly Lys Leu Met Glu Val His Gly Asp Tyr Thr Ala Glu Asp Val
   225                 230                 235                 240
   Gly Val Lys Val Asp Arg Pro Ala Asp Glu Thr Met Val Glu Glu Pro
                   245                 250                 255
   Thr Glu Ile Ile Gly
               260
<210> 3
   <211> 1169
   <212> DNA
   <213> Saccharum officinarum
<400> 3
   aagatgaagc ctttgtcatg gtgcatgcta aagatgctga ggctgagaag ttgagggatg     60
   aaccatgaca aaggcttcat ctttctcgac ctgaatcctg tccacattcc ccttcagcat    120
   cttcaattca gcctcgatca ttttcttctt aagcaccccg ccgtcgttct cttcctgcat    180
   ccccgcccca ttccctagcg tcgcccccct cgccgccgca cggacgcagc gacgagctct    240
   cgcagcagca atggcggttg gcaagaataa gcgtatctcc aagggcaaga agggaggcaa    300
   gaagaagacc gtggatccgt tcagcaaaaa ggattggtat gatatcaagg ctccgtcggt    360
   cttcagcgtg cgcaacatcg gcaagaccct ggtctccagg acacagggca ccaagattgc    420
   ctctgagggt ttaaagcaca gagtatttga ggtctccttg gctgatcttc agagtgatga    480
   agaccaggcg tacaggaaga tcagacttcg tgcagaggat gtacaaggga gaaatgttct    540
   cacaaacttc tggggtatga gcttcaccac cgacaagctc cgttcacttg tgaagaagtg    600
   gcagacgctt attgaggctc atgttgatgt caagaccacc gataactata tgctgcggct    660
   gttctgcatt gggttcacca agaggcggcc caatcaagtg aagcgcactt gctatgctca    720
   agcaagccaa atcagacaga ttcgtcggaa gatgactgaa atcatgagca accaagcttc    780
   aacttgtgat ctgaaagagc tcgtgtccaa gttcatccct gaggtcattg gaaaggaaat    840
   cgagaaagcc acctctagca tattcccctt gcaaaatgtc ttcatccgca aggtgaagat    900
   cctgaaagca ccaaagttcg acattggaaa gctcatggag gtccatggtg actatgccaa    960
   ggaggatgtt ggtgtcaaga tggacaggcc tgctgaaggc gacgaggcca tgggaggaca   1020
   ggaggttgct gcagctgagt gattagtctc actgtttacg tccgagttag agctgccata   1080
   tttccttgaa acacttagga acactttttt tgagagtctg acatgtggtg gcttcgattc   1140
   tccttgaaaa tttgcagcat gggaaatgt                                     1169
<210> 4
   <211> 263
   <212> PRT
   <213> Saccharum officinarum
<400> 4
   Met Ala Val Gly Lys Asn Lys Arg Ile Ser Lys Gly Lys Lys Gly Gly
   1               5                   10                  15
   Lys Lys Lys Thr Val Asp Pro Phe Ser Lys Lys Asp Trp Tyr Asp Ile
               20                  25                  30
   Lys Ala Pro Ser Val Phe Ser Val Arg Asn Ile Gly Lys Thr Leu Val
           35                  40                  45
   Ser Arg Thr Gln Gly Thr Lys Ile Ala Ser Glu Gly Leu Lys His Arg
       50                  55                  60
   Val Phe Glu Val Ser Leu Ala Asp Leu Gln Ser Asp Glu Asp Gln Ala
   65                  70                  75                      80
   Tyr Arg Lys Ile Arg Leu Arg Ala Glu Asp Val Gln Gly Arg Asn Val
                   85                  90                  95
   Leu Thr Asn Phe Trp Gly Met Ser Phe Thr Thr Asp Lys Leu Arg Ser
               100                 105                 110
   Leu Val Lys Lys Trp Gln Thr Leu Ile Glu Ala His Val Asp Val Lys
           115                 120                 125
   Thr Thr Asp Asn Tyr Met Leu Arg Leu Phe Cys Ile Gly Phe Thr Lys
       130                 135                 140
   Arg Arg Pro Asn Gln Val Lys Arg Thr Cys Tyr Ala Gln Ala Ser Gln
   145                 150                 155                 160
   Ile Arg Gln Ile Arg Arg Lys Met Thr Glu Ile Met Ser Asn Gln Ala
                   165                 170                 175
   Ser Thr Cys Asp Leu Lys Glu Leu Val Ser Lys Phe Ile Pro Glu Val
               180                 185                 190
   Ile Gly Lys Glu Ile Glu Lys Ala Thr Ser Ser Ile Phe Pro Leu Gln
           195                 200                 205
   Asn Val Phe Ile Arg Lys Val Lys Ile Leu Lys Ala Pro Lys Phe Asp
       210                 215                 220
   Ile Gly Lys Leu Met Glu Val His Gly Asp Tyr Ala Lys Glu Asp Val
   225                 230                 235                 240
   Gly Val Lys Met Asp Arg Pro Ala Glu Gly Asp Glu Ala Met Gly Gly
                   245                 250                 255
   Gln Glu Val Ala Ala Ala Glu
               260
<210> 5
   <211> 51
   <212> DNA
   <213> Artificial sequence
<220>
   <223> primer prm02255
<400> 5
   ggggacaagt ttgtacaaaa aagcaggctt cacaatggct gtcgggaaga a 51
<210> 6
   <211> 49
   <212> DNA
   <213> Artificial sequence
<220>
   <223> primer prm02256
<400> 6
   ggggaccact ttgtacaaga aagctgggtc ctaagctccg atgatttct 49